(19) Europäisches Patentamt
European Patent Office
Office européen des brevets

(11) Veröffentlichungsnummer : **0 364 403 B1**

# (12) EUROPÄISCHE PATENTSCHRIFT

(45) Veröffentlichungstag der Patentschrift :
**24.03.93 Patentblatt 93/12**

(51) Int. Cl.[5] : **C07C 309/39,** C07C 309/32, C07C 309/59, C07C 303/32, D06L 3/12

(21) Anmeldenummer : **89810751.1**

(22) Anmeldetag : **04.10.89**

## (54) Distyrylbiphenylverbindungen.

(30) Priorität : **13.10.88 CH 3829/88**

(43) Veröffentlichungstag der Anmeldung :
**18.04.90 Patentblatt 90/16**

(45) Bekanntmachung des Hinweises auf die Patenterteilung :
**24.03.93 Patentblatt 93/12**

(84) Benannte Vertragsstaaten :
**CH DE FR GB IT LI**

(56) Entgegenhaltungen :
**FR-A- 1 583 595**

(73) Patentinhaber : **CIBA-GEIGY AG**
**Klybeckstrasse 141**
**CH-4002 Basel (CH)**

(72) Erfinder : **Weber, Kurt, Dr.**
**Rennweg 98**
**CH-4052 Basel (CH)**
Erfinder : **Eckhardt, Claude, Dr.**
**16, Rue des Jonquilles**
**F-68400 Riedisheim (FR)**

EP 0 364 403 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Distyrylbiphenylverbindungen, Verfahren zu deren Herstellung unter Einschluss neuer Zwischenprodukte, sowie deren Verwendung.

Die Verwendung von optischen Aufhellern in flüssigen Waschmitteln ist allgemein bekannt. Sie ziehen während der Behandlung auf das Waschgut auf und führen durch ihre spezielle Lichtabsorption/Emissionseigenschaft zu einer Erhaltung bzw. Verbesserung des ursprünglichen weissen Aspektes.

Dieser Effekt ist aber auch verantwortlich für das Auftreten von hellen Flecken wenn Textilgewebe z.B. bei einer Vorbehandlung direkt mit dem flüssigen Waschmittel in Kontakt gerät. In der EP-A-167 205 wird zur Lösung dieses Problems daher vorgeschlagen, spezifische Stilbentriazolyl-, -triazin- oder Distyrylbiphenyl-Aufheller zu verwenden.

Ueberraschenderweise kann die Bildung heller Flecken, bei ausgezeichneter Aufhellwirkung, durch neue Verbindungen der Formel

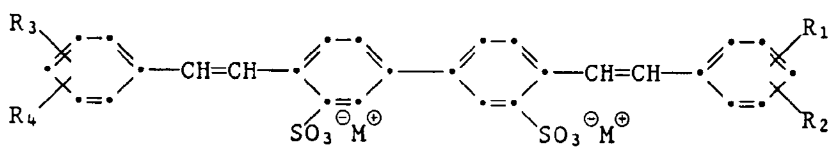

(I)

verhindert werden.

Im einzelnen bedeutet:

$R_1$ bzw. $R_3$ unabhängig voneinander Wasserstoff, Halogen, $C_1$-$C_4$-Alkyl oder CN,

$R_2$ bzw. $R_4$ unabhängig voneinander Halogen, $C_1$-$C_4$-Alkyl oder CN

$M^{\oplus}$ salzbildendes Kation.

Als Halogene kommen vor allem Fluor, Chlor und Brom in Frage, insbesondere jedoch Chlor.

Als $C_1$-$C_4$-Alkylreste kommen unverzweigte und verzweigte Alkylreste wie der Methyl-, Ethyl-, n- und iso-Propyl, n-, sec- und tert.-Butylrest in Betracht. Diese $C_1$-$C_4$-Alkylreste können ihrerseits substituiert sein mit z.B. Aryl-(Phenyl-, Naphthyl-), $C_1$-$C_4$-Alkyl-, $C_1$-$C_4$-Alkoxy-, OH- oder CN-Gruppen.

Salzbildende Kationen $M^{\oplus}$ sind z.B. Alkalimetall-, Ammonium- oder Aminsalzione. Unter Aminsalzionen sind solche der Formel $H^{+}NR_8R_9R_{10}$ bevorzugt, in denen $R_8$, $R_9$ und $R_{10}$ unabhängig voneinander Wasserstoff, Alkyl, Alkenyl, Hydroxyalkyl, Cyanoalkyl, Halogenalkyl oder Phenylalkyl bedeuten oder worin $R_8$ und $R_9$ zusammen die Ergänzung zu einem 5-7-gliedrigen gesättigten Stickstoffheterocyclus darstellen, der noch zusätzlich ein Stickstoff- oder Sauerstoffatom als Ringglied enthalten kann, beispielsweise einen Piperidin-, Piperazin, Pyrrolidin-, Imidazolin- oder Morpholinring, während $R_{10}$ für Wasserstoff steht.

Bevorzugte Distyrylbiphenylverbindungen der Formel (I) sind solche in denen das Kation $M^{\oplus}$ ein Alkalimetall-, Ammonium-, oder Aminion ist, wobei aus praktischen Erwägungen Kalium und Natrium eine besondere Bedeutung haben.

Insbesondere sind symmetrische Verbindungen der Formel (I) von Interesse, und vor allem solche, bei denen $R_1$ und $R_3$ Wasserstoff ist. Besonders zu erwähnen hierbei sind die $C_1$-$C_4$-Alkyl- vor allem $CH_3$ oder Cl-Reste $R_2$ und $R_4$. Hervorzuheben sind Verbindungen der Formel (II)

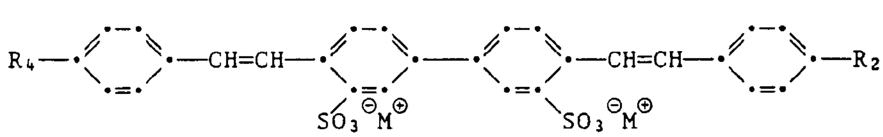

(II)

worin $R_2$ und $R_4$ die Bedeutung aus Formel (I) haben.

Von praktischem Interesse sind parasubstituierte Verbindungen wie z.B.

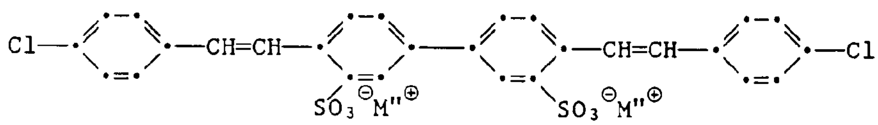
(100)

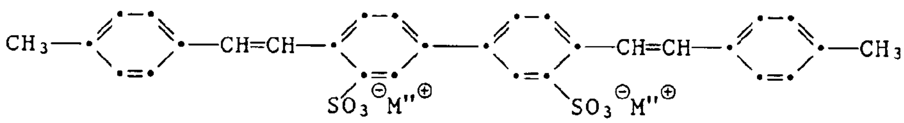
(101)

worin M″⊕ Natrium oder Kalium bedeutet.

Die Herstellung der Distyrylbiphenylverbindungen erfolgt vor allem auf zwei Wegen (A oder B), wobei Weg A zur Herstellung von symmetrischen und unsymmetrischen Verbindungen dient und Weg B vorzugsweise zur Herstellung der symmetrischen Verbindung beschritten wird.

A:

Ausgehend von mindestens einem Mol einer Verbindung der Formel

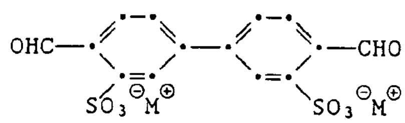
(X)

und einem Mol einer Verbindung der Formel

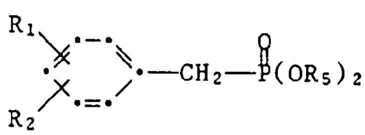
(XI)

sowie einem Mol einer Verbindung der Formel

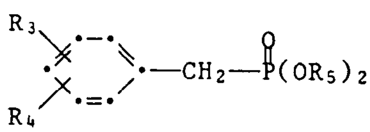
(XII)

gelangt man zu Verbindungen der Formel (I) durch Umsetzung in einem polaren Lösungsmittel in Gegenwart starker Basen.

B:

In Gegenwart von Pd-Kohle setzt man zwei Mol einer Verbindung der Formel

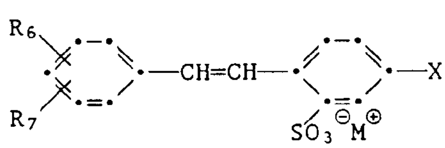
(XX)

mit einer Base in einem polaren Lösungsmittel zu symmetrischen Verbindungen der Formel

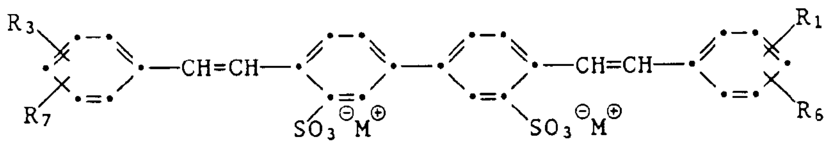
(III)

um.

Die Ausgangsstilbenverbindungen der Formel (XX)

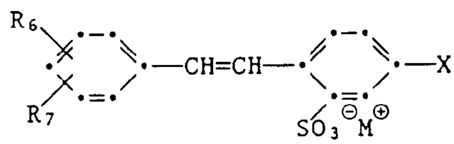
(XX)

sind neu; darin bedeuten $R_6$ beziehungsweise $R_7$ Halogen, Wasserstoff, $C_1$-$C_4$-Alkyl oder CN, X Halogen und $M^{\oplus}$ ein salzbildendes Kation, ausgenommen die Verbindungen in denen $R_6$ Wasserstoff, $R_7$ Chlor in para-Stellung zum Stilbenrest und X Chlor sind.

Von besonderer Bedeutung sind solche in denen $M^{\oplus}$ ein Alkalimetall-, Ammonium- oder Aminion ist und unter diesen besonders jene in denen $M^{\oplus}$ Natrium oder Kalium ist.

Im Hinblick auf die Umsetzung von (XX) zu Verbindungen der Formel (I) sind solche Verbindungen bevorzugt, in denen X Chlor ist.

Insbesondere Verbindungen der Formel (XX) worin $R_7$ Wasserstoff ist sollen erwähnt werden.

Besonders bevorzugt bei den monosubstituierten Verbindungen der Formel

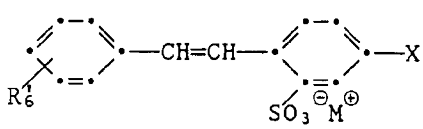
(XXI)

sind diejenigen in denen $R_6'$ $C_1$-$C_4$-Alkyl, vor allem $CH_3$, oder CN bedeutet.

Insbesondere sind Verbindungen der Formel (XXI) von Interesse, bei denen X Chlor, $R_6'$ $C_1$-$C_4$-Alkyl und $M'^{\oplus}$ Alkalimetall-, Ammonium- oder Aminion bedeuten.

Eine besondere Bedeutung kommt der Verbindung

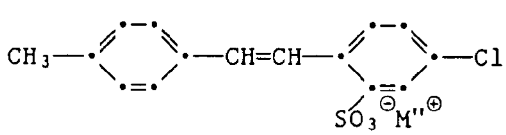
(200)

zu, wobei $M''^{\oplus}$ Natrium oder Kalium bedeutet.

Die neuen Verbindungen der Formel (XX) lassen sich in an sich bekannter Weise durch die Reaktion von Benzylphosphonsäureestern der Formel

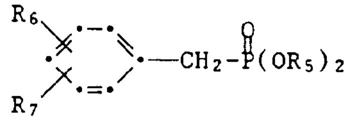
(XXX)

mit Aldehyden der Formel

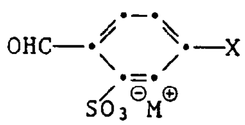 (XXXI)

in einem polaren Lösungsmittel und in Gegenwart einer starken Base herstellen.

$R_1$, $R_3$, $R_6$ beziehungsweise $R_7$ kann dabei unabhängig voneinander Halogen, Wasserstoff, $C_1$-$C_4$-Alkyl oder CN, $R_2$ bzw. $R_4$ unabhängig voneinander Halogen, $C_1$-$C_4$-Alkyl oder CN, $R_5$ $C_1$-$C_4$-Alkyl und X Halogen sein. Für $M^{\oplus}$, die möglichen Halogene und die Alkylreste gilt das oben gesagte.

Als polare Lösungsmittel kommen z.B. Alkohole wie Methanol, Ethanol, Isopropanol, Butanol, Glykole, Glykolether wie 2-Methoxyethanol, Hexanole, Cyclohexanol und Cyclooctanol, ferner Ether wie Diisopropylether, Tetrahydrofuran und Dioxan sowie Dimethylsulfoxyd, Formamid und N-Methylpyrrolidon in Betracht. Besonders geeignet sind polare organische Lösungsmittel wie Dimethylformamid und Dimethylsulfoxyd. Auch in wässriger Lösung lassen sich einige der Umsetzungen durchführen.

Als starke Basen kommen vor allem die Hydroxyde, Amide und Alkoholate (vorzugsweise solche primärer, 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen wie z.B. Phenyllithium oder stark basische Amine (einschliesslich Ammoniumbasen) z.B. Trialkylammoniumhydroxyde, mit Erfolg verwendet werden.

Die Temperatur, bei welcher diese Umsetzungen durchgeführt werden, kann in weiten Grenzen schwanken. Sie wird bestimmt:

α) durch die Beständigkeit des verwendeten Lösungsmittels gegenüber den Reaktionsteilnehmern, insbesondere gegenüber den stark basischen Alkaliverbindungen,

β) durch die Reaktivität der Kondensationspartner und

γ) durch die Wirksamkeit der Kombination Lösungsmittel-Base als Kondensationsmittel.

In der Praxis kommen hiernach im allgemeinen Temperaturen zwischen etwa 10° und 100°C in Betracht, insbesondere, wenn Dimethylformamid oder Dimethylsulfoxyd als Lösungsmittel verwendet werden. Der Temperatur-Vorzugsbereich liegt bei 20° bis 70°C.

Verbindungen der Formel (I) finden Verwendung zur Aufhellung von Textilmaterialien, wobei Polyamide, Wolle und Baumwolle besonders hervorzuheben sind und von Papier.

Eine weitere Verwendung besteht in der Zubereitung von Waschmittelformulierungen, festen, gelartigen und flüssigen. Hierbei zeichnen sich die Verbindungen der Formel (I) durch sehr gute Weisseffekte bei geringem Spottingverhalten aus.

Die erfindungsgemässen Waschpulver enthalten z.B. die bekannten Mischungen von Waschaktivsubstanzen wie beispielsweise Seife in Form von Schnitzeln und Pulver, Synthetika, lösliche Salze von Sulfonsäurehalbestern höherer Fettalkohole, höher und/oder mehrfach alkylsubstituierter Arylsulfonsäuren, Sulfocarbonsäureester mittlerer bis höherer Alkohole, Fettsäureacylaminoalkyl- oder -aminoarylglycerinsulfonate, Phosphorsäureester von Fettalkoholen usw. Als Aufbaustoffe, sogenannte "Builders", kommen z.B. Alkalisalze der Carboxymethylcellulose und andere "Soilredepositionsinhibitoren", ferner Alkalisilikate, Alkalicarbonate, Alkaliborate, Alkaliperborate, Alkalipercarbonate, Nitrilotriessigsäure, Ethylendiaminotetraessigsäure, Schaumstabilisatoren wie Alkanolamide höherer Fettsäuren, in Betracht. Ferner können in den Waschmitteln beispielsweise enthalten sein; antistatische Mittel, rückfettende Hautschutzmittel wie Lanolin, Antimikrobika, Parfüme und andere optische Aufheller, sowie Bleichmittel wie Perborat, allein oder in Kombination mit sogenannten Aktivatoren, wie z.B. TAED, TAGU, NOBS etc. oder Percarbonat oder Persäuren; oder Photobleichmittel wie z.B. sulfonierte Zn oder Al Phthalocyanine.

Beispielsweise enthalten erfindungsgemässe Waschpulver 10-50 Gew.-% einer anionischen, nichtionischen, semipolaren, ampholytischen oder/und zwitterionischen oberflächenaktiven Substanz, 0-80 % eines alkalischen Gerüststoffsalzes, 0-30 % eines Bleichmittels oder Bleichsystems und gegebenenfalls weitere übliche Waschmittelbestandteile, beispielsweise solche, die vorstehend erwähnt sind.

Als oberflächenaktive Substanzen in besagten Waschpulvern kommen beispielsweise auch wasserlösliche Alkylbenzolsulfonate, Alkylsulfate, ethoxylierte Alkylethersulfate, Paraffinsulfonate, α-Olefinsulfonate, α-Sulfocarbonsäuren, deren Salze und Ester, Alkylglycerylethersulfonate, Fettsäuremonoglyceridsulfate oder -sulfonate, Alkylphenolpolyethoxyethersulfate, 2-Acyloxyalkansulfonate, β-Alkyloxyalkansulfonate, Seifen, ethoxylierte Fettalkohole, Alkylphenole, Polypropoxyglykole, Polypropoxy-ethylendiamine, Aminoxide, Phosphinoxide, Sulfoxide, aliphatische sekundäre und tertiäre Amine, aliphatische quaternäre Ammonium-, Phosphoniumund Sulfoniumverbindungen oder Mischungen der genannten Substanzen in Betracht.

Beispiele für alkalische Gerüststoffsalze, die z.B. in einer Menge von 10-60 Gew.-% in den erfindungsgemässen Waschpulvern vorhanden sein können, sind unter anderen;
wasserlösliche Alkalimetallcarbonate, -borate, -phosphate, -polyphosphate, -bicarbonate und -silicate, wasserlösliche Aminopolycarboxylate, Phytate, Polyphosphonate und -carboxylate, sowie wasserunlösliche Aluminiumsilicate.

Vorteilhaft wird das Waschgranulat durch Sprühtrocknung hergestellt.

Verbindungen der Formel (I), unter Einschluss der Verbindungen in denen $R_2$ und/oder $R_4$ Wasserstoff sind, eignen sich besonders zur Verwendung als optische Aufheller in flüssigen Waschmitteln.

Unter Flüssigwaschmitteln sind bekannte und handelsübliche Waschmittel wie sie beispielsweise in der EP-A-167 205 oder US-4 507 219 beschrieben werden, zu verstehen.

Insbesondere enthalten die Flüssigwaschmittel neben 0,01-1 %, vorzugsweise 0,05-0,2 %, eines optischen Aufhellers der Formel (III) 1 bis 60 % anionische, nichtionische, zwitterionische und gegebenenfalls kationische Tenside und 25 bis 65 % vorzugsweise 40 bis 55 % Wasser. Im einzelnen enthält das Waschmittel neben dem optischen Aufheller 3 bis 50 % vorzugsweise 15 bis 25 % anionische Tenside, 2 bis 30 % vorzugsweise 4 bis 15 % nichtionische Tenside, 3 bis 30 % vorzugsweise 5 bis 20 % gegebenenfalls ethoxylierte ($C_{10}$-$C_{14}$)-Fettsäuren wie Kaprin-, Laurin-, Myristin-, Kokusnuss- und Palmkernsäure sowie Mischungen davon, 1 bis 25 % vorzugsweise 1 bis 10 % Waschmittelaufbaustoffe sowie gegebenenfalls 1 bis 10% vorzugsweise 1 bis 5 % zwitterionische Tenside, 0,5 bis 3 % vorzugsweise 0,7 bis 2 % quaternäre Ammonium-, Amin- oder Aminoxid-Tenside, und 1 bis 10 % übliche Waschmittelzusätze wie zum Beispiel Enzyme, Enzymstabilisatoren, Antioxidantien, Konservierungs- und Desinfektionsmittel, Duft- und Farbstoffe, Komplexbildner bzw. Sequestriermittel und Lösungsmittel.

Brauchbare Tenside werden z.B. in der US-4 285 841, US-3 929 678, US-4 284 532 und GB-2 041 986 beschrieben. Insbesondere werden die in der EP-A-167 205 als bevorzugt bezeichneten Tenside eingesetzt. Vor allem verwendet man jedoch als anionische Tenside gegebenenfalls ethoxylierte $C_{10}$-$C_{18}$-Alkylsulfate z.B. in Form der Triethanolaminsalze, $C_{10}$-$C_{15}$-Alkylbenzolsulfonate oder Mischungen davon und als nichtionische Tenside Kondensationsprodukte aus einem Mol ($C_{10}$-$C_{15}$)-Fettalkohol mit 3 bis 8 Mol Ethylenoxid.

Als Waschmittelaufbaustoffe kommen die in der US-4 321 165 und US-4 284 532 erwähnten vorzugsweise polycarboxylierte Verbindungen wie zum Beispiel Zitronensäure in Betracht.

Bei der Verwendung von Verbindungen der Formel

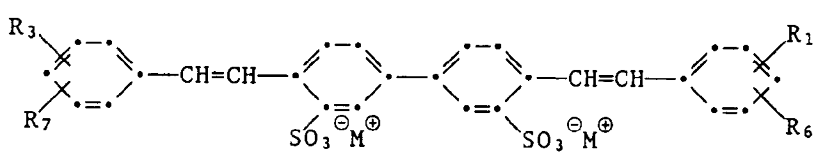

(III)

wobei $R_1$, $R_3$, $R_6$ und $R_7$ unabhängig voneinander Halogen, Wasserstoff, $C_1$-$C_4$-Alkyl oder CN und $M^{\oplus}$ ein salzbildendes Kation bedeutet, zeichnen sich die flüssigen Waschmittel durch ausgezeichnete Aufhelleigenschaften, Lagerbeständigkeit und geringes Spottingverhalten aus.

Bevorzugte Distyrylbiphenylverbindungen der Formel (III) sind solche in denen das Kation $M^{\oplus}$ ein Alkalimetall-, Ammonium-, oder Aminion ist, wobei aus praktischen Erwägungen Kalium und Natrium eine besondere Bedeutung haben.

Insbesondere sind symmetrische Verbindungen der Formel (III) von Interesse, und vor allem solche, bei denen $R_1$ und $R_3$ Wasserstoff ist (VIa). Besonders zu erwähnen hierbei sind die $C_1$-$C_4$-Alkyl- vor allem $CH_3$ oder Cl-Reste $R_6$ und $R_7$. Hervorzuheben sind Verbindungen der Formel (IIa)

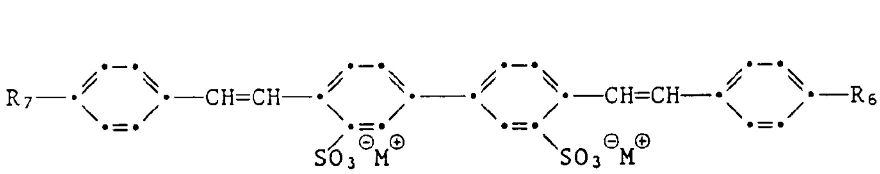

(IIa)

worin $R_6$ und $R_7$ die Bedeutung aus Formel (III) haben.

Von praktischem Interesse sind Verbindungen wie z.B. solche der Formel

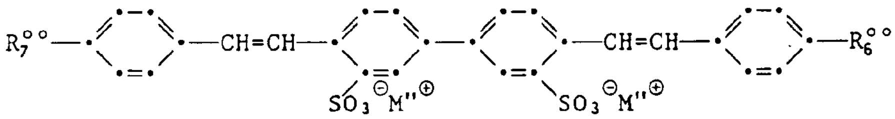

(VIIIa)

worin $R_6^{oo}$ und $R_7^{oo}$ Chlor bzw. $CH_3$ und $M''^{\oplus}$ Natrium oder Kalium bedeutet.

Die folgenden Beispiele dienen zu Erläuterung der Erfindung; Teile bedeuten Gewichtsteile und Prozente Gewichtsprozente; der Fleckentest wird folgendermassen durchgeführt:

a) Aufheller/Waschmittel-Formulierung:

0,1 % (100 % Aktivsubstanz) optischer Aufheller oder Aufhellergemisch werden in einem flüssigen Waschmittel gelöst. 0,6 g dieses Aufheller enthaltenden Waschmittels (A) wird mit 400 ml Wasser (10°-12° dH) bei einer Temperatur von 30°C verdünnt (Waschflotte B).

b) Ein 20 g Stück gebleichtes Baumwollgewebe wird auf einem Spannrahmen befestigt.

c) Auf eine vormarkierte, runde Fläche (5 cm Durchmesser) dieses Baumwollgewebes werden mit einer Pipette 0,6 ml der Waschmittellösung (A) gleichmässig aufgetragen, nach 30 Sekunden Einwirkzeit in die vorbereitete Waschflotte (B) gegeben und während 15 Minuten bei 30°C gewaschen. Anschliessend wird mit kaltem Wasser gespült und bei 70°C getrocknet.

d) Der Unterschied des Weissgrades nach Ganz zwischen der Auftragsfläche und der Umgebung ist ein Mass für das sogenannte Spotting-Verhalten (Bildung von hellen Flecken) und wird bei einfacher Textillage mit einem RFC3-Photometer von Zeiss bestimmt.

Beispiel 1:

8,9 g 4,4'-Bisformyl-diphenyl-3,3'-disulfonsäure, Kaliumsalz und 10,5 g 1-Diethylphosphonomethyl-4-chlorbenzol werden in 120 ml Dimethylformamid unter Rühren bei 40°C vorgelegt. Dann werden 2,7 g Kaliumhydroxidpulver (Gehalt: 89 %) zugegeben, auf 65°C erhitzt und während 4 Stunden gerührt. Nach dem Abkühlen auf Raumtemperatur werden 120 ml destilliertes Wasser zugegeben, auf 90°C erhitzt, klärfiltriert, das Filtrat abgekühlt auf Raumtemperatur und das auskristallisierte Produkte abgenutscht. Das Nutschgut wird aus einer Mischung von 50 ml destilliertem Wasser und 50 ml Dimethylformamid umkristallisiert. Nach dem Trocknen unter Vakuum erhält man 1,5 g der Verbindung der Formel

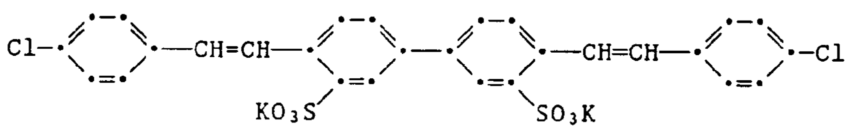

(103)

als grünstichig gelbes Kristallpulver UV - Spektrum: $\lambda_{max}$ = 357 nm, $\varepsilon$ = 69007 (aufgenommen in $DMF/H_2O$ 1:1)

Beispiel 2:

Gemäss der Vorgehensweise in Beispiel 1 können folgende Verbindungen erhalten werden:

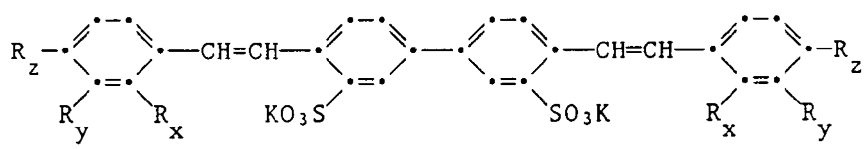

| Nr. | $R_x$ | $R_y$ | $R_z$ | UV ($DMF/H_2O$ 1:1) | |
|---|---|---|---|---|---|
| | | | | $\lambda_{max}$ (nm) | ε |
| (104) | H | CN | H | 353 | 73 376 |
| (105) | CN | H | H | 353 | 56 880 |
| (106) | $CH_3$ | H | H | 353 | 63 207 |
| (107) | H | Cl | H | 353 | 66 292 |
| (108) | H | Cl | Cl | 357 | 69 630 |
| (109) | Cl | H | Cl | 356 | 70 418 |

Beispiel 3:

Zu einer Suspension von 13,2 g Kaliumhydroxydpulver (Gehalt 89 %) in 40 ml Dimethylformamid wird unter Rühren und Ausschluss von Luft eine Suspension von 2,7 g (0,007 mol) 4,4′-Bisformyl-diphenyl-3,3′-disulfosäure, Natriumsalz, und 2,96 g (0,012 mol)1-Diethylphosphonomethyl-benzol innert 5 Minuten eingetragen. Es wird noch 5 Stunden bei 40-45°C nachgerührt und in 350 ml destilliertes Wasser von 70°C gegeben. In diese Lösung gibt man 150 g Kochsalz und 150 g Eis, lässt über Nacht rühren, nutscht ab und wäscht mit 500 ml 20%iger Sole nach. Der Nutschkuchen wird in 140 ml destilliertem Wasser heiss gelöst, mit 2 g Aktivkohle versetzt, filtriert und auskristallisieren gelassen. Die Kristallmasse wird abgenutscht und bei 80-85°C in Vakuum getrocknet. Man erhältt so 0,23 g der Substanz der Formel

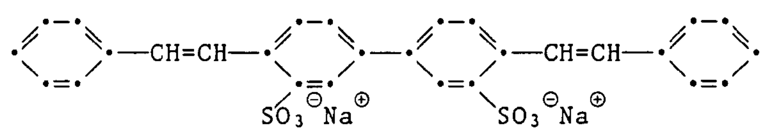

(110)

als blassgelbes Kristallpulver.
Schmelzpunkt: über 300°C.

Beispiel 4:

45,6 g (0,12 mol) 4-Chlor-benzaldehyd-2-sulfosäure, Kaliumsalz (Gehalt 70 %) und 30,5 g (0,12 mol) 1-Diethylphosphono-methyl-4-methyl-benzol werden bei 40°C in 300 ml Dimethylformamid unter Rühren vorgelegt. Es werden 12,5 g Kaliumhydroxyd eingetragen und 15 Stunden bei 40°C nachgerührt. Dann werden 350 ml Wasser zugegeben, mit Essigsäure neutralisiert, klärfiltriert, das Filtrat zur Trockene eingedampft und der Rückstand aus 200 ml Wasser umkristallisiert. Man erhält 27 g der Verbindung der Formel

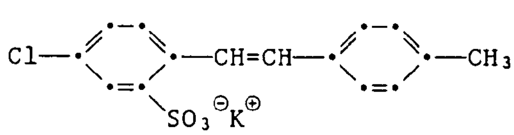

(201)

als weisses Kristallpulver.
Schmelzpunkt: über 300°C.

Beispiel 5:

17,3 g (0,05 mol) 4-Chlor-4'-methyl-stilben-2-sulfosäure, Kaliumsalz, gemäss Beispiel 4 und 2,8 g Kaliumhydroxyd werden unter Rühren in 200 ml entsalztem Wasser gelöst. Nach Zugabe von 1 g 5%iger Pd-Kohle werden bei 85-90°C innerhalb 4 Stunden 80,4 ml Methanol/Wasser (1:1) zugetropft, anschliessend rührt man noch 15 Stunden bei 85-90°C. Im Anschluss an die Klärfiltration unter Zusatz eines Filtrationshilfsmittels wird zur Trockene eingedampft. Das Rohprodukt wird über eine Kieselgel-Säule mit 11,5 cm Durchmesser chromatographiert. Als Eluiermittel verwendet man: 100 Volumenteile Isopropanol + 35 Volumenteile Toluol + 20 Volumenteile Ammoniak (25 %) und 100 Volumenteile Methylcellosolve + 80 Volumenteile entsalztes Wasser + 20 Volumenteile Ammoniak (25 %). Das so erhaltene gelbe Produkt kristallisiert man aus 250 ml Wasser + 50 ml Methylcellosolve unter Zugabe von etwas Kaliumhydroxyd und Aktivkohle um. Nach dem Trocknen im Vakuum bei 110°C erhält man 1,5 g der Verbindung der Formel

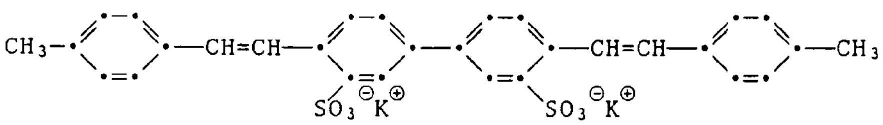 (111)

als gelbes Kristallpulver.
Schmelzpunkt: über 300°C.

Beispiel 6:

Zu einer Papiermasse aus 100 Teilen gebleichter Cellulose wird im Holländer 2 Teile Harzleim gegeben. Nach 10 Minuten fügt man zuerst 0,2 Teile der Verbindung gemäss Beispiel 5, in 20 Teilen Wasser gelöst, zu, dann nach weiteren 15 Minuten 3 Teile Aluminiumsulfat. Die so behandelte Masse gelangt dann über eine Mischbütte auf eine Papiermaschine, auf welcher das Papier in bekannter Weise hergestellt wird.

Das so gewonnene Papier zeigt einen hohen Aufhelleffekt.

Beispiel 7:

100 Teile Baumwollstoff werden im Flottenverhältnis von 1:20 während 20 Minuten bei 60°C in einem Bad, enthaltend 0,1 Teile Verbindung gemäss Beispiel 5 und 2,5 g/l Glaubersalz, behandelt, wobei nach 5 Minuten weitere 2,5 g/l Glaubersalz zugegeben werden. Darauf wird gespült und getrocknet.

Es resultiert ein starker Aufhelleffekt.

Beispiel 8:

1 g der Verbindung gemäss Beispiel 5 wird in 1 l weichem Wasser, das 2 g Polyphosphat sowie 5 ml Essigsäure (80%ig) enthält, gelöst. Ein Stück Polyamide 6 (®Perlon Webtrikot) wird bei einer Flottenaufnahme von 110 % mit diesem Bad kalt foulardiert, und anschliessend während 40 Sekunden bei 190°C thermofixiert.

Nach dieser Behandlung weist das Textilmaterial einen hohen Weissgrad auf.

Beispiel 9:

100 Teile Polyamid 66 (Nylon Webtrikot) werden bei einem Flottenverhältnis von 1:20 in weichem Wasser, das 0,1 Teile der verbinddung gemäss Beispiel 5, sowie 3 g/l stabilisiertes Hydrosulfit und 1 ml/l Essigsäure (80%ig) enthält, innerhalb von 30 Minuten von 40°C auf 98°C aufgeheizt. Nach 30 Minuten bei 98°C wird innerhalb von 15 Minuten auf 40°C abgekühlt, kalt gespült und in einem Trockenschrank bei 60°C getrocknet.

Es resultiert ein hoher, brillanter Weissgrad.

Beispiel 10:

Durch Sprühtrocknung eines Slurry bestehend aus 1 Teil Wasser und 1 Teil Waschmittel folgender Zusammensetzung:

8,4 g lineares Dodecylbenzolsulfonat
3,1 g Talgalkohol-tetradecan-ethylen-glykolether (14 AeO)
3,7 g Na-Seife (vorwiegend aus Behen-Säure und $C_{14}$-$C_{20}$-Säuren)
45,8 g Na-Tripolyphosphat
7,9 g Na-Silikat
2,0 g Mg-Silikat
1,2 g Carboxymethylcellulose
0,2 g Ethylendiamintetraacetat
22,2 g Na-Sulfat
0,1 g der Verbindung gemäss Beispiel 5

wird ein Waschmittelgranulat mit einer Restfeuchte von ca. 5 % hergestellt.

4 Teile dieses Waschmittelgranulates werden in 1000 Teilen Wasser (12° dH) bei einer Temperatur von 40°C aufgelöst. Fünf Stücke gebleichte Baumwolle (entsprechend je 10 Teilen) werden in diesem Bad bei 40°C während 15 Minuten gewaschen, dann unter kaltem, fliessendem Wasser gespült und in einer Schwingmaschine bei einer Tourenzahl von ca. 1000 Touren/Minute während 30 Sekunden zentrifugiert. Dieser Waschprozess wird 3 mal durchgeführt, dann werden die Baumwollstücke getrocknet und nach der Methode von Ganz mit einem Photometer (RFC 3 von Zeiss) auf ihren Weissgrad bestimmt.

Es resultieren sehr hohe Weisseffekte, in der Grössenordnung von 200 Weisseinheiten.

Beispiel 11:

Der Fleckentest wird mit einem optischen Aufheller der Formel

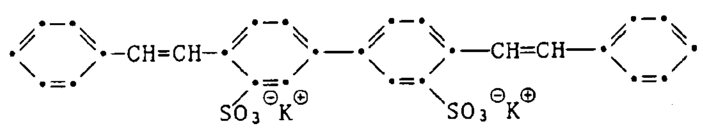

(112)

und einem flüssigen Waschmittel enthaltend

15 Teile $C_{11}$-$C_{13}$ Alkylbenzolsulfonat
14 Teile $C_{14}$-$C_{15}$ Polyethoxyfettalkohol (7 Ethylenoxid)
10 Teile Seife
9 Teile Ethanol
4 Teile Na-Citrat
5 Teile Triethanolamin
43 Teile Wasser

durchgeführt. Die 0,1 Teile des optischen Aufhellers lassen sich leicht in dieser Formulierung als klare und über mehrere Monate stabile Lösung einarbeiten.

Der Fleckentest zeigt nur eine geringe Bildung von hellen Flecken, obwohl 0,1 Teile des genannten optischen Aufhellers in dem hier beschriebenen Waschmittel eingearbeitet, in Waschversuchen sehr hohe Aufhelleffekte liefert, und dies bereits bei niedrigen Waschtemperaturen. Wird z.B. gebleichte Baumwolle mit 3 g des obigen Waschmittels pro Liter Waschflotte, bei einem Flottenverhältnis von 1:20 und einer Temperatur von 30°C während 15 Minuten gewaschen, erhält man Weissgrade - spektrophotometrisch nach der Methode von Ganz gemessen - von ca. 140 nach der ersten Wäsche oder ca. 175 nach 5 Waschzyklen.

Beispiel 12:

Verfährt man wie im Beispiel 11 beschrieben, verwendet aber anstelle des dort genannten optischen Aufhellers einen solchen das Beispiels 5, so ergeben sich Weisseffekte von ca. 150 nach der ersten, bzw. ca. 195 nach der fünften Wäsche bei 30°C, oder ca. 155 nach der ersten, bzw. ca. 195 nach der fünften Wäsche bei einer Waschtemperatur von 60°C, sowie eine noch geringere Bildung heller Flecken.

Auch wenn bei jedem Waschgang die Trocknung der aufgehellten Baumwolle mit Aussetzen am Tageslicht bis zu jeweils 200 Langley geschieht, was etwa dem Trocknen "im Freien" in europäischen Regionen entspricht, ist nur ein sehr geringer, kaum sichtbarer Weissgradverlust gegenüber der nicht belichteten Probe festzustellen.

## Patentansprüche

1. Distyrylbiphenylverbindungen der Formel

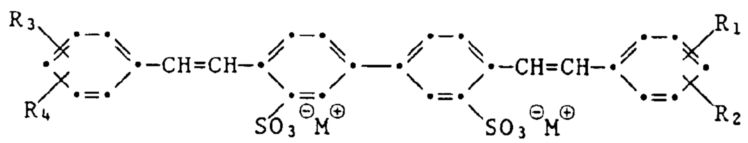

(I)

worin bedeutet:
$R_1$ bzw. $R_3$ unabhängig voneinander Halogen, Wasserstoff, $C_1$-$C_4$-Alkyl oder CN,
$R_2$ bzw. $R_4$ unabhängig voneinander Halogen, $C_1$-$C_4$-Alkyl oder CN und $M^{\oplus}$ ein salzbildendes Kation.

2. Distyrylbiphenylverbindungen gemäss Anspruch 1, dadurch gekennzeichnet, dass diese symmetrisch sind.

3. Distyrylbiphenylverbindungen gemäss Anspruch 1 der Formel

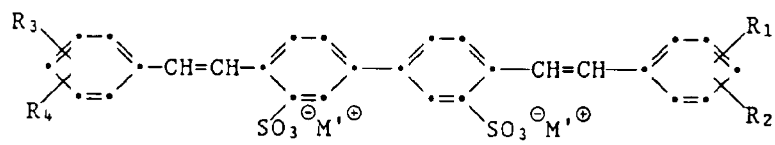

(IV)

worin $M'^{\oplus}$ ein Alkalimetall-, Ammonium- oder Aminion bedeutet und $R_1$ bis $R_4$ die in Anspruch 1 angegebene Bedeutung haben.

4. Distyrylbiphenylverbindungen gemäss Anspruch 1 der Formel

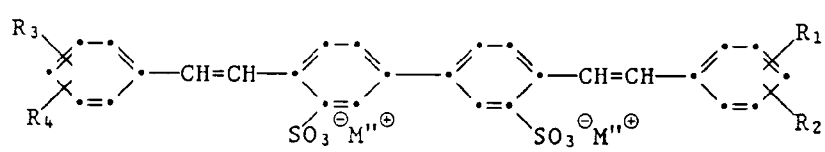

(V)

worin $M''^{\oplus}$ Natrium oder Kalium bedeutet, und $R_1$ bis $R_4$ die in Anspruch 1 angegebene Bedeutung haben.

5. Distyrylbiphenylverbindungen gemäss Anspruch 1 der Formel

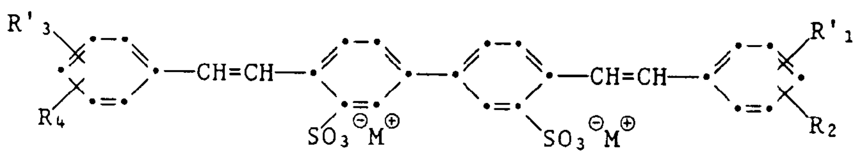

(VI)

worin $R'_1$ und $R'_3$ Wasserstoff und $R_2$, $R_4$ und $M^{\oplus}$ die im Anspruch 1 angegebene Bedeutung haben.

6. Distyrylbiphenylverbindungen gemäss Anspruch 1 der Formel

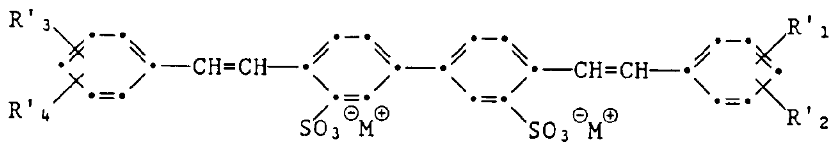

(VII)

worin $R'_2$ und $R'_4$ $C_1$-$C_4$-Alkyl oder Cl, $R'_1$ und $R'_3$ Wasserstoff und $M^{\oplus}$ ein salzbildendes Kation bedeutet.

**7.** Distyrylbiphenylverbindungen gemäss Anspruch 1 der Formel

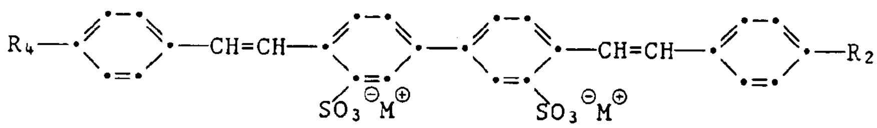

(II)

worin $R_2$, $R_4$ und $M^{\oplus}$ die in Anspruch 1 angegebene Bedeutung haben.

**8.** Distyrylbiphenylverbindungen der Formel

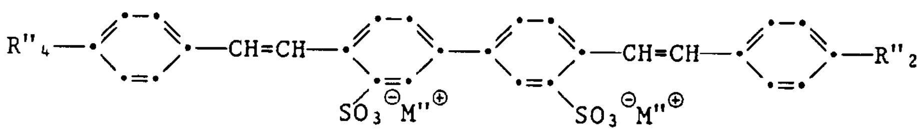

(VIII)

worin $R''_2$ und $R''_4$ Chlor bzw. $CH_3$ und $M''^{\oplus}$ Natrium oder Kalium bedeutet.

**9.** Verbindungen der Formel

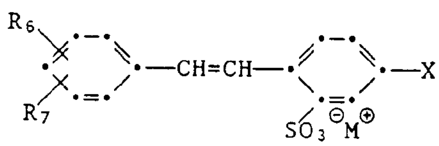

(XX)

worin bedeutet $R_6$ beziehungsweise $R_7$ unabhängig voneinander Halogen, Wasserstoff, $C_1$-$C_4$-Alkyl oder CN, X Halogen und $M^{\oplus}$ein salzbildendes Kation, ausgenommen die Verbindungen in denen $R_6$ Wasserstoff, $R_7$ Chlor in para-Stellung zum Stilbenrest und X Chlor sind.

**10.** Verbindungen gemäss Anspruch 9 der Formel

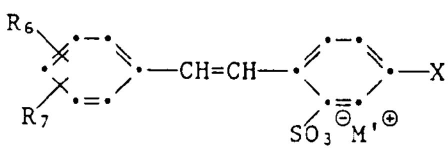

(XXII)

worin $M'^{\oplus}$ Ammonium-, Amin- oder ein Alkaliion bedeutet und $R_6$, $R_7$ und X die in Anspruch 9 angegebene Bedeutung haben.

**11.** Verbindungen gemäss Anspruch 9 der Formel

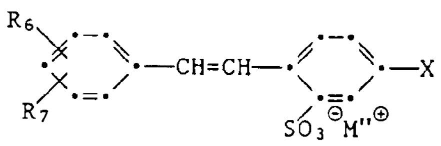

(XXIII)

worin $M''^{\oplus}$ Natrium oder Kalium bedeutet und $R_6$, $R_7$ und X die in Anspruch 9 angegebene Bedeutung haben.

**12.** Verbindungen gemäss Anspruch 9 der Formel

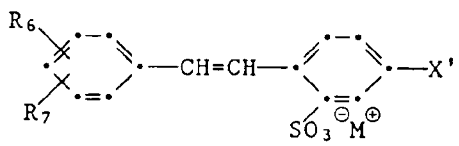

(XXIV)

worin X' Chlor bedeutet und $R_6$, $R_7$ und $M^{\oplus}$ die in Anspruch 9 angegebene Bedeutung haben.

**13.** Verbindungen gemäss Anspruch 9 der Formel

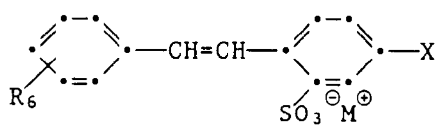

(XXI)

worin $R_6$, X und $M^{\oplus}$ die in Anspruch 9 angegebne Bedeutung haben.

**14.** Verbindungen gemäss Anspruch 13 der Formel

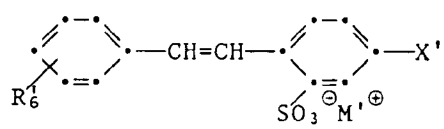

(XXV)

worin $R_6'$ $C_1$-$C_4$-Alkyl, X' Chlor und $M'^{\oplus}$ ein Alkali-, Ammonium- oder Aminion bedeutet.

**15.** Verbindungen gemäss Anspruch 13 der Formel

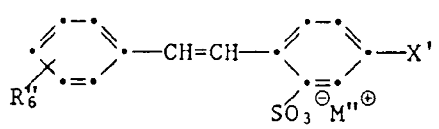

(XXVI)

worin $R_6''$ Methyl, X' Chlor und $M''^{\oplus}$ Natrium oder Kalium bedeutet.

**16.** Verfahren zur Herstellung von Verbindungen der Formel (XX) gemäss Anspruch 9

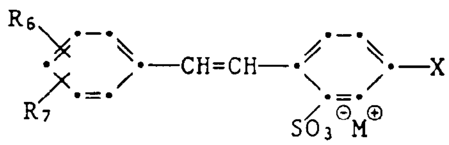

(XX)

dadurch gekennzeichnet, dass man ein Mol einer Verbindung der Formel

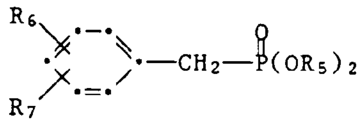

(XXX)

mit mindestens einem Mol einer Verbindung der Formel

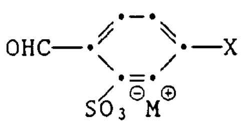

(XXXI)

in Anwesenheit einer starken Base und eines polaren Lösungsmittels umsetzt, wobei $R_6$ beziehungsweise $R_7$ unabhängig voneinander Halogen, Wasserstoff, $C_1$-$C_4$-Alkyl oder CN, $R_5$ $C_1$-$C_4$-Alkyl, X Halogen und $M^{\oplus}$ein salzbildendes Kation, ausgenommen die Verbindungen in denen $R_5$ Wasserstoff, $R_7$ Chlor in para-Stellung zum Stilben- bzw. Phosphonatrest und X Chlor ist.

**17.** Verfahren zur Herstellung von Distyrylbiphenylverbindungen gemäss Anspruch 1, dadurch gekennzeichnet, dass man mindestens ein Mol einer Verbindung der Formel

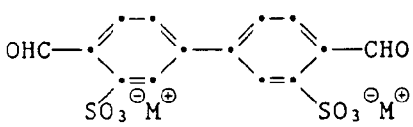

(X)

mit einem Mol einer Verbindung der Formel

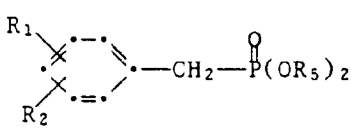

(XI)

und einem Mol einer Verbindung der Formel

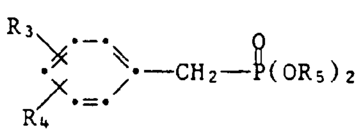

(XII)

wobei $R_1$, $R_2$, $R_3$ und $R_4$ und $M^{\oplus}$die in Anspurch 1 angegebene Bedeutung haben und $R_5$ einen Alkylrest mit 1-4 C-Atomen bedeutet, in Anwesenheit einer starken Base und eines polaren Lösungsmittels umsetzt.

**18.** Verfahren zur Herstellung von symmetrischen Distyrylbiphenylverbindungen der Formel

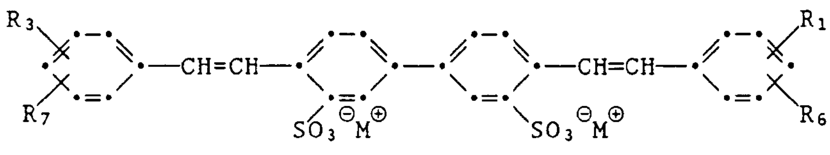

(III),

dadurch gekennzeichnet, dass man zwei Mol einer Verbindung der Formel (XX)

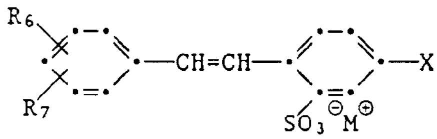

(XX)

in Gegenwart einer Base und Pd-Kohle in einem polaren Lösungsmittel umsetzt, wobei $R_1$, $R_3$, $R_6$ und $R_7$ unabhängig voneinander Halogen, Wasserstoff, $C_1$-$C_4$-Alkyl oder CN, $M^{\oplus}$ein salzbildendes Kation und X Halogen bedeutet.

**19.** Verwendung der Distyrylbiphenylverbindungen gemäss Anspruch 1 zum optischen Aufhellen von Textilmaterial.

**20.** Verwendung der Distyrylbiphenylverbindungen gemäss Anspruch 1 zum optischen Aufhellen von Baumwolle, Polyamid und Wolle.

**21.** Verwendung der Distyrylbiphenylverbindungen gemäss Anspruch 1 zum optischen Aufhellen von Papier.

**22.** Verwendung der Distyrylbiphenylverbindungen gemäss Anspruch 1 in festen Waschmitteln, als optische Aufheller.

**23.** Verwendung der Distyrylbiphenylverbindungen der Formel

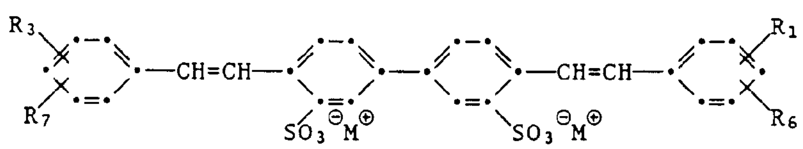

(III)

in flüssigen Waschmitteln, als optischer Aufheller, wobei $R_1$, $R_3$, $R_6$ und $R_7$ unabhängig voneinander Halogen, Wasserstoff, $C_1$-$C_4$-Alkyl oder CN und $M^{\oplus}$ein salzbildendes Kation bedeutet.

**24.** Verwendung von symmetrisch substituierten Distyrylbiphenylverbindungen der Formel

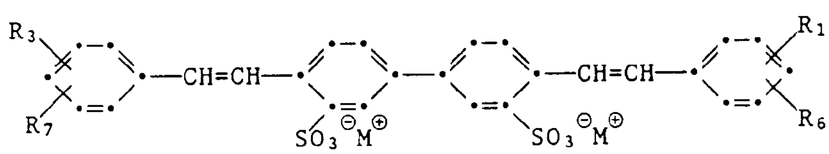

(III)

in flüssigen Waschmitteln, als optischer Aufheller, wobei $R_1$, $R_3$, $R_6$ und $R_7$ unabhängig voneinander Halogen, Wasserstoff, $C_1$-$C_4$-Alkyl oder CN und $M^{\oplus}$ein salzbildendes Kation bedeutet.

**25.** Verwendung von Distyrylbiphenylverbindungen gemäss Anspruch 23 der Formel

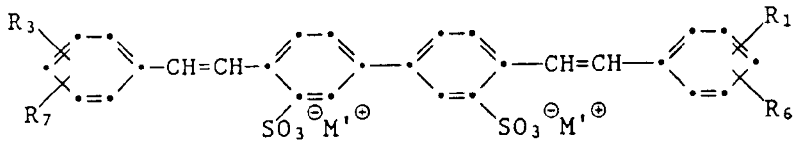
(IVa)

in flüssigen Waschmitteln, als optischer Aufheller, wobei $M'^{\oplus}$ ein Alkalimetall-, Ammonium- oder Aminion bedeutet und $R_1$, $R_3$, $R_6$ und $R_7$ die in Anspruch 23 angegebene Bedeutung haben.

**26.** Verwendung von Distyrylbiphenylverbindungen gemäss Anspruch 23 der Formel

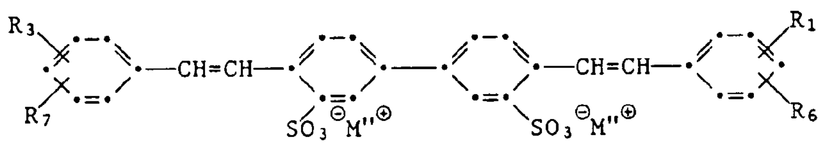
(Va)

in flüssigen Waschmitteln, als optischer Aufheller, worin $M''^{\oplus}$ Natrium oder Kalium bedeutet, und $R_1$, $R_3$, $R_6$ und $R_7$ die in Anspruch 23 angegebene Bedeutung haben.

**27.** Verwendung von Distyrylbiphenylverbindungen gemäss Anspruch 23 der Formel

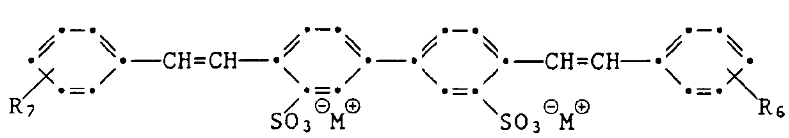
(VIa)

in flüssigen Waschmitteln, als optischer Aufheller, $R_6$, $R_7$ und $M^{\oplus}$ die im Anspruch 23 angegebene Bedeutung haben.

**28.** Verwendung von Distyrylbiphenylverbindungen gemäss Anspruch 23 der Formel

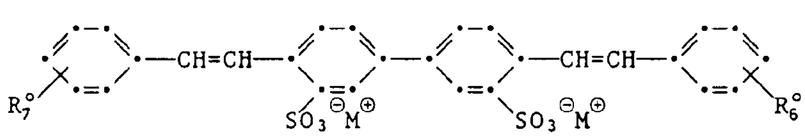
(VIIa)

in flüssigen Waschmitteln, als optischer Aufheller, worin $R_6^{\circ}$ und $R_7^{\circ}$ $C_1$-$C_4$-Alkyl oder Cl und $M^{\oplus}$ ein salzbildendes Kation bedeutet.

**29.** Verwendung von Distyrylbiphenylverbindungen gemäss Anspruch 23 der Formel

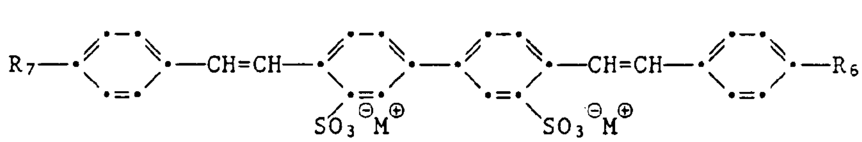
(IIa)

in flüssigen Waschmitteln, als optischer Aufheller, worin $R_6$, $R_7$ und $M^{\oplus}$ die in Anspruch 24 angegebene

Bedeutung haben.

**30.** Verwendung von Distyrylbiphenylverbindungen gemäss Anspruch 23 der Formel

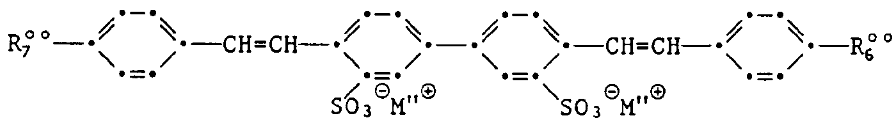

(VIIIa)

worin $R_6^{\circ\circ}$ und $R_7^{\circ\circ}$ Chlor bzw. $CH_3$ und $M''^{\oplus}$ Natrium oder Kalium bedeutet.

## Claims

**1.** A distyrylbiphenyl compound of formula

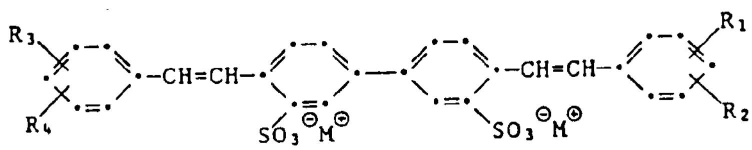

(I)

in which
$R_1$ and $R_3$ are each independently of the other halogen, hydrogen, $C_1$-$C_4$alkyl or CN,
$R_2$ and $R_4$ are each independently of the other halogen, $C_1$-$C_4$alkyl or CN, and
$M^{\oplus}$ is a salt-forming cation.

**2.** A distyrylbiphenyl compound according to claim 1 which is symmetrical.

**3.** A distyrylbiphenyl compound according to claim 1 of formula

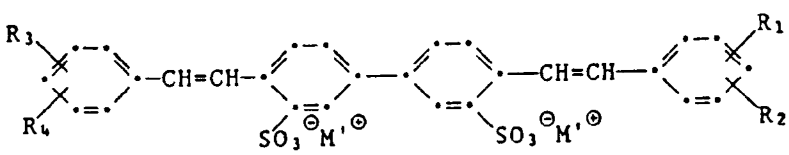

(IV)

in which $M'^{\oplus}$ is an alkali metal ion, an ammonium ion or an amine ion, and $R_1$ to $R_4$ are as defined in claim 1.

**4.** A distyrylbiphenyl compound according to claim 1 of formula

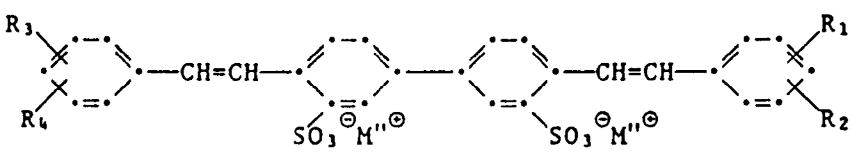

(V)

in which $M''^{\oplus}$ is sodium or potassium, and $R_1$ to $R_4$ are as defined in claim 1.

**5.** A distyrylbiphenyl compound according to claim 1 of formula

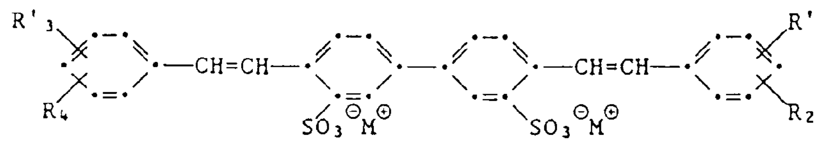 (VI)

in which $R'_1$ and $R'_3$ are hydrogen, and $R_2$, $R_4$ and $M^{\oplus}$ are as defined in claim 1.

6. A distyrylbiphenyl compound according to claim 1 of formula

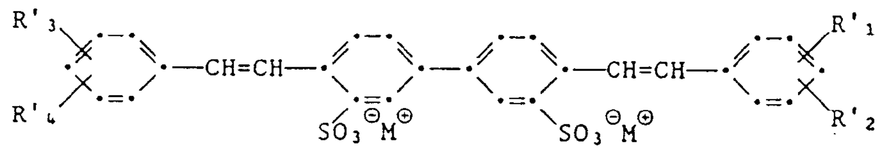 (VII)

in which $R'_2$ and $R'_4$ are $C_1$-$C_4$alkyl or Cl; $R'_1$ and $R'_3$ are hydrogen, and $M^{\oplus}$ is a salt-forming cation.

7. A distyrylbiphenyl compound according to claim 1 of formula

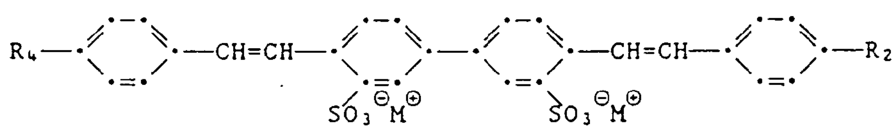 (II)

in which $R_2$, $R_4$ and $M^{\oplus}$ are as defined in claim 1.

8. A distyrylbiphenyl compound of formula

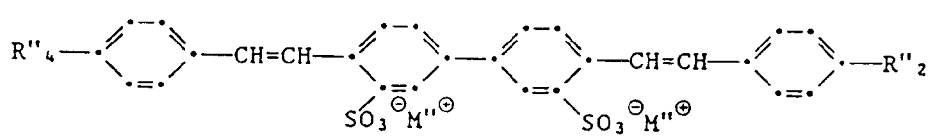 (VIII)

in which $R''_2$ and $R''_4$ are chlorine or $CH_3$, and $M''^{\oplus}$ is sodium or potassium.

9. A compound of formula

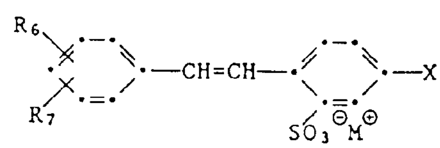 (XX)

in which $R_6$ and $R_7$ are each independently of the other halogen, hydrogen, $C_1$-$C_4$alkyl or CN, X is halogen and $M^{\oplus}$ is a salt-forming cation, excluding the compounds in which $R_6$ is hydrogen, $R_7$ is chlorine in para-position to the stilbene radical and X is chlorine.

10. A compound according to claim 9 of formula

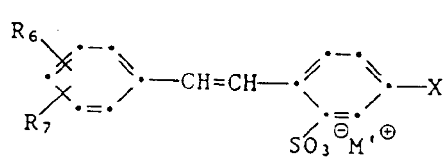 (XXII)

in which $M'^{\oplus}$ is an ammonium ion, an amine ion or an alkali metal ion, and $R_6$, $R_7$ and X are as defined in

claim 9.

**11.** A compound according to claim 9 of formula

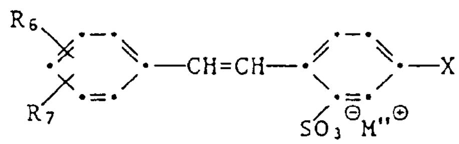

(XXIII)

in which $M''^{\oplus}$ is sodium or potassium, and $R_6$, $R_7$ and X are as defined in claim 9.

**12.** A compound according to claim 9 of formula

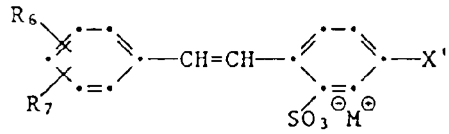

(XXIV)

in which X' is chlorine and $R_6$, $R_7$ and $M^{\oplus}$ are as defined in claim 9.

**13.** A compound according to claim 9 of formula

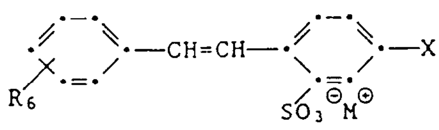

(XXI)

in which $R_6$, X and $M^{\oplus}$ are as defined in claim 9.

**14.** A compound according to claim 13 of formula

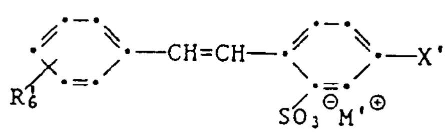

(XXV)

in which $R'_6$ is $C_1$-$C_4$alkyl, X' is chlorine, and $M'^{\oplus}$ is an alkali metal ion, an ammonium ion or an amine ion.

**15.** A compound according to claim 13 of formula

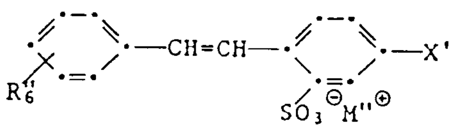

(XXVI)

in which $R''_6$ is methyl, X' is chlorine and $M''^{\oplus}$ is sodium or potassium.

**16.** A process for the preparation of a compound of formula (XX) according to claim 9

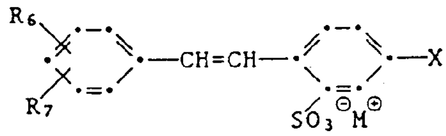

(XX)

which comprises reacting 1 mol of a compound of formula

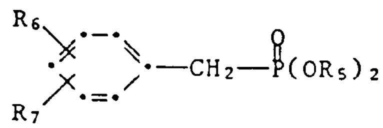

(XXX)

with at least 1 mol of a compound of formula

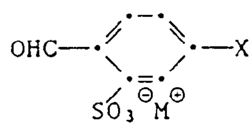

(XXXI)

in the presence of a strong base and of a polar solvent, where $R_6$ and $R_7$ are each independently of the other halogen, hydrogen, $C_1$-$C_4$alkyl or CN, $R_5$ is $C_1$-$C_4$alkyl, X is halogen, and $M^{\oplus}$ is a salt-forming cation, excluding the compounds in which $R_6$ is hydrogen, $R_7$ is chlorine in para-position to the stilbene or phosphonate radical, and X is chlorine.

**17.** A process for the preparation of a distyrylbiphenyl compound according to claim 1, which comprises reacting at least 1 mol of a compound of formula

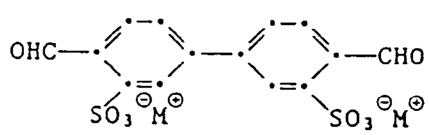

(X)

with 1 mol of a compound of formula

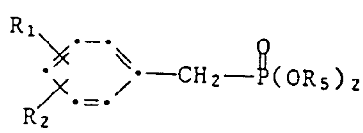

(XI)

and 1 mol of a compound of formula

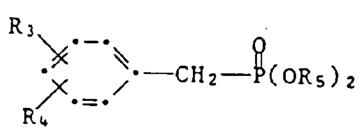

(XII)

where $R_1$, $R_2$, $R_3$, $R_4$ and $M^{\oplus}$ are as defined in claim 1, and $R_5$ is an alkyl radical having 1-4 C atoms in the presence of a strong base and a polar solvent.

**18.** A process for the preparation of a symmetrical distyrylbiphenyl compound of formula

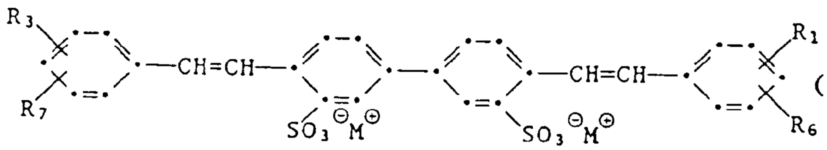

(III)

which comprises reacting 2 mol of a compound of formula (XX)

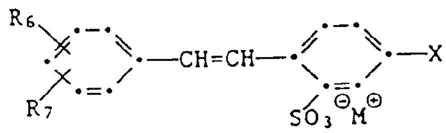

(XX)

in the presence of a base and Pd/C and in a polar solvent, where $R_1$, $R_3$, $R_6$ and $R_7$ are each independently of the other halogen, hydrogen, $C_1$-$C_4$alkyl or CN, $M^{\oplus}$ is a salt-forming cation, and X is halogen.

**19.** Use of a distyrylbiphenyl compound according to claim 1 for whitening textile material.

**20.** Use of a distyrylbiphenyl compound according to claim 1 for whitening cotton, polyamide and wool.

**21.** Use of a distyrylbiphenyl compound according to claim 1 for whitening paper.

**22.** Use of a distyrylbiphenyl compound according to claim 1 in solid detergent compositions as fluorescent whitening agent.

**23.** Use of a distyrylbiphenyl compound of formula

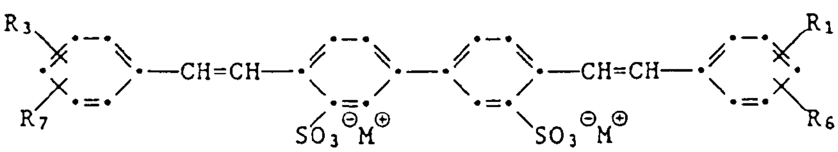

(III)

where $R_1$, $R_3$, $R_6$ and $R_7$ are each independently of the other halogen, hydrogen, $C_1$-$C_4$alkyl or CN, and $M^{\oplus}$ is a salt-forming cation, as fluorescent whitening agent in liquid detergent compositions.

**24.** Use of a symmetrical substituted distyrylbiphenyl compound of formula

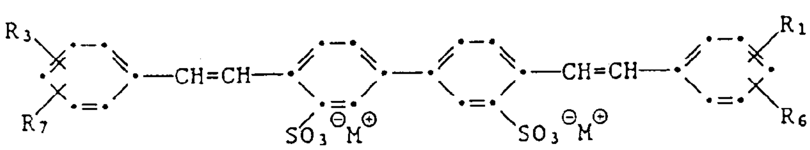

(III)

where $R_1$, $R_3$, $R_6$ and $R_7$ are each independently of the other halogen, hydrogen, $C_1$-$C_4$alkyl or CN, and $M^{\oplus}$ is a salt-forming cation, as fluorescent whitening agent in liquid detergent compositions.

**25.** Use of a distyrylbiphenyl compound according to claim 23 of formula

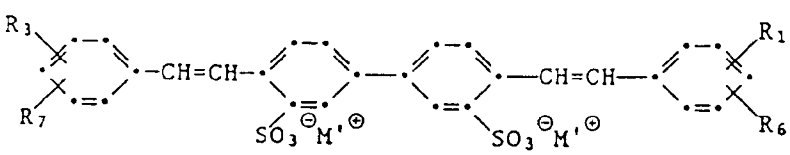

(IVa)

in which $M'^{\oplus}$ is an alkali metal ion, an ammonium ion or an amine ion, and $R_1$, $R_3$, $R_6$ and $R_7$ are as defined in claim 23, as fluorescent whitening agent in liquid detergent compositions.

**26.** Use of a distyrylbiphenyl compound according to claim 23 of formula

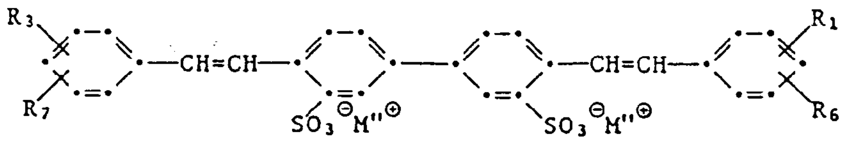

(Va)

in which $M''^{\oplus}$ is sodium or potassium, and $R_1$, $R_3$, $R_6$ and $R_7$ are as defined in claim 23, as fluorescent whitening agent in liquid detergent compositions.

**27.** Use of a distyrylbiphenyl compound according to claim 23 of formula

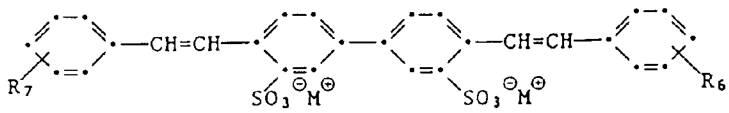

(VIa)

in which $R_6$, $R_7$ and $M^{\oplus}$ are as defined in claim 23, as fluorescent whitening agent in liquid detergent compositions.

**28.** Use of a distyrylbiphenyl compound according to claim 23 of formula

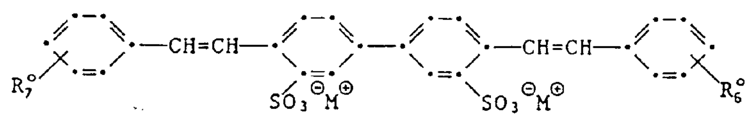

(VIIa)

in which $R_6^{\circ}$ and $R_7^{\circ}$ are $C_1$-$C_4$alkyl or Cl, and $M^{\oplus}$ is a salt-forming cation, as fluorescent whitening agent in liquid detergent compositions.

**29.** Use of a distyrylbiphenyl compound according to claim 23 of formula

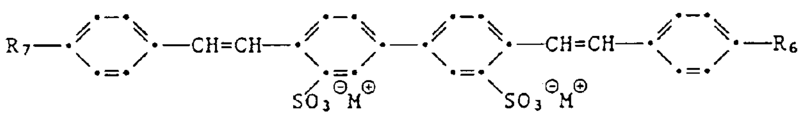

(IIa)

in which $R_6$, $R_7$ and $M^{\oplus}$ are as defined in claim 24, as fluorescent whitening agent in liquid detergent compositions.

**30.** Use of a distyrylbiphenyl compound according to claim 23 of formula

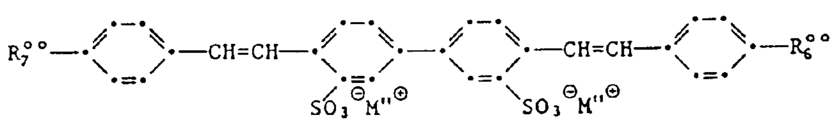

(VIIIa)

wherein $R_6^{\circ\circ}$ and $R_7^{\circ\circ}$ are chlorine or $CH_3$, and $M''^{\oplus}$ is sodium or potassium.

## Revendications

**1.** Composés distyrylbiphényle de formule

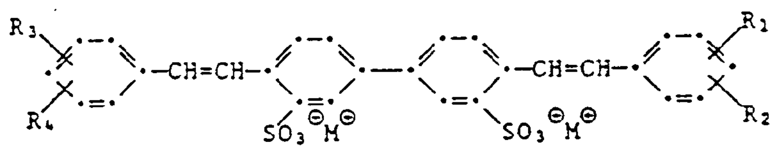

(I)

dans laquelle
$R_1$ et $R_3$ représentent chacun, indépendamment l'un de l'autre, un atome d'hydrogène ou d'halogène ou un groupe alkyle en $C_{1-4}$ ou CN, et
$R_2$ et $R_4$ représentent chacun, indépendamment l'un de l'autre, un atome d'halogène ou un groupe alkyle en $C_{1-4}$ ou CN, et
$M^+$ représente un cation formant un sel.

**2.** Composés distyrylbiphényle conformes à la revendication 1, **caractérisé** en ce qu'ils sont symétriques.

**3.** Composés distyrylbiphényle conformes à la revendication 1, de formule

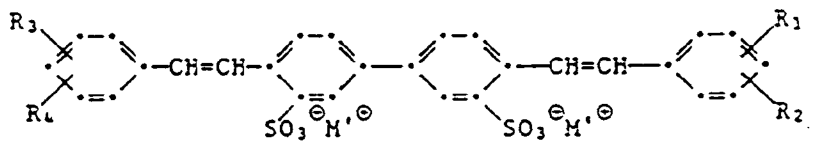

(IV)

dans laquelle $M'^+$ représente un ion de métal alcalin, un ion ammonium ou un ion d'amine, et $R^1$ à $R_4$ ont les significations indiquées dans la revendication 1.

**4.** Composés distyrylbiphényle conformes à la revendication 1, de formule

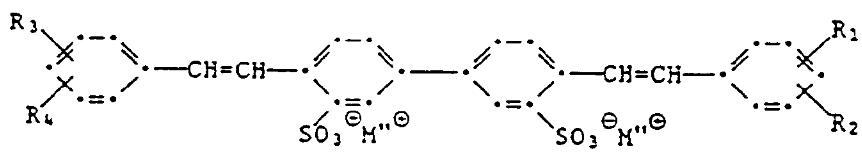

(V)

dans laquelle $M''^+$ représente un ion de sodium ou de potassium, et $R_1$ à $R_4$ ont les significations indiquées dans la revendication 1.

**5.** Composés distyrylbiphényle conformes à la revendication 1, de formule

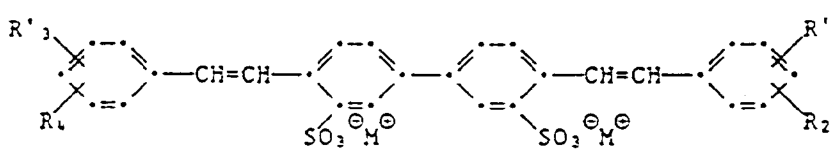

(VI)

dans laquelle $R'_1$ et $R'_3$ représentent des atomes d'hydrogène, et $R_2$, $R_4$ et $M^+$ ont les significations indiquées dans la revendication 1.

6. Composés distyrylbiphényle conformes à la revendication 1, de formule

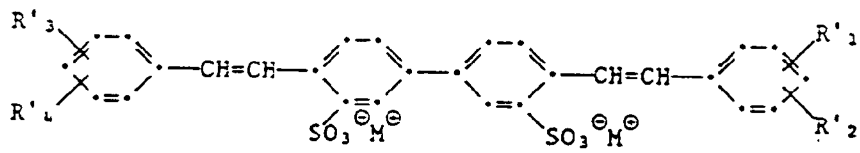

(VII)

dans laquelle $R'_2$ et $R'_4$ représentent des groupes alkyle en $C_{1-4}$ ou des atomes de chlore, $R'_1$ et $R'_3$ représentent des atomes d'hydrogène et $M^+$ représente un cation formant un sel.

7. Composés distyrylbiphényle conformes à la revendication 1, de formule

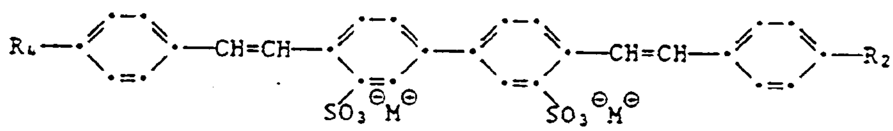

(II)

dans laquelle $R_2$, $R_4$ et $M^+$ ont les significations indiquées dans la revendication 1.

8. Composés distyrylbiphényle de formule

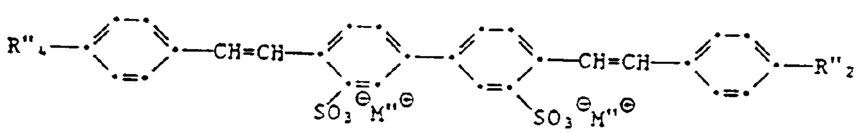

(VIII)

dans laquelle $R''_2$ et $R''_4$ représentent des atomes de chlore ou des groupes méthyle et $M''^+$ représente un ion de sodium ou de potassium.

9. Composés de formule

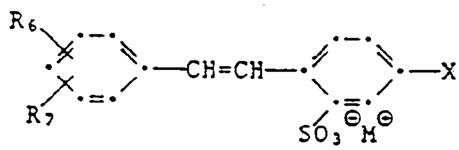

(XX)

dans laquelle $R_6$ et $R_7$, indépendamment l'un de l'autre, représentent chacun un atome d'hydrogène ou d'halogène ou un groupe alkyle en $C_{1-4}$ ou CN, X représente un atome d'halogène et $M^+$ représente un cation formant un sel, à l'exception de composés dans lesquels $R_6$ représente un atome d'hydrogène, $R_7$ représente un atome de chlore en position para par rapport au reste de stilbène, et X représente un atome de chlore.

**10.** Composés conformes à la revendication 9, de formule

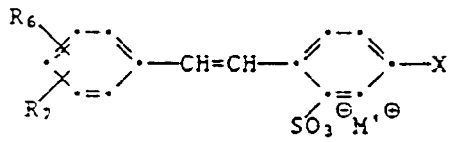

(XXII)

dans laquelle $M'^{+}$ représente un ion ammonium, un ion d'amine ou un ion de métal alcalin, et $R_6$, $R_7$ et X ont les significations indiquées dans la revendication 9.

**11.** Composés conformes à la revendication 9, de formule

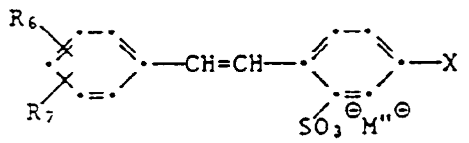

(XXIII)

dans laquelle $M''^{+}$ représente un ion de sodium ou de potassium et $R_6$, $R_7$ et X ont les significations indiquées dans la revendication 9.

**12.** Composés conformes à la revendication 9, de formule

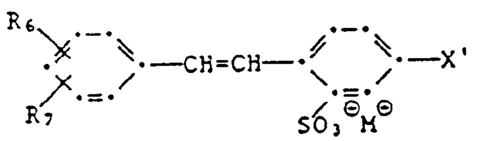

(XXIV)

dans laquelle X' représente un atome de chlore et $R_6$, $R_7$ et $M^+$ ont les significations indiquées dans la revendication 9.

**13.** Composés conformes à la revendication 9, de formule

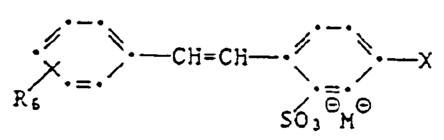

(XXI)

dans laquelle $R_6$, X et $M^+$ ont les significations indiquées dans la revendication 9.

**14.** Composés conformes à la revendication 13, de formule

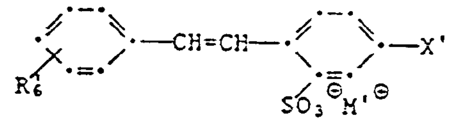

(XXV)

dans laquelle $R'_6$ représente un groupe alkyle en $C_{1-4}$, X' représente un atome de chlore et $M'^{+}$ représente un ion de métal alcalin, un ion ammonium ou un ion d'amine.

**15.** Composés conformes à la revendication 13, de formule

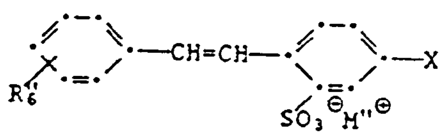

(XXVI)

dans laquelle $R''_6$ représente un groupe méthyle, X' représente un atome de chlore et $M'^{+}$ représente un ion de sodium ou de potassium.

**16.** Procédé de préparation de composés de formule (XX) conforme à la revendication 9

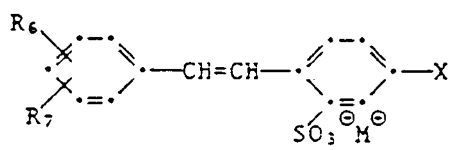

(XX)

caractérisé en ce que l'on fait réagir une mole d'un composé de formule

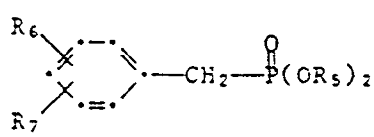

(XXX)

avec au moins une mole d'un composé de formule

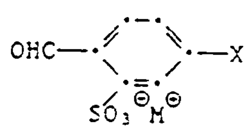

(XXXI)

en présence d'une base forte et d'un solvant polaire, $R_6$ et $R_7$ représentant chacun, indépendamment l'un de l'autre, un atome d'hydrogène ou d'halogène ou un groupe alkyle en $C_{1-4}$ ou CN, $R_5$ représentant un groupe alkyle en $C_{1-4}$, X représentant un atome d'halogène et $M^{+}$ représentant un cation formant un sel, excepté les composés dans lesquels $R_6$ représente un atome d'hydrogène, $R_7$ représente un atome de chlore en position para par rapport au reste de stilbène ou de phosphonate, et X représente un atome de chlore.

**17.** Procédé de préparation de composés distyrylbiphényle conformes à la revendication 1, caractérisé en ce que l'on fait réagir au moins une mole d'un composé de formule

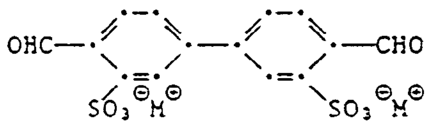 (X)

avec une mole d'un composé de formule

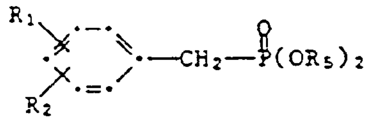 (XI)

et une mole d'un composé de formule

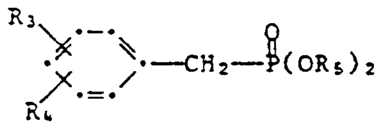 (XII)

dans lesquelles formules $R_1$, $R_2$, $R_3$ et $R_4$ et $M^+$ ont les significations indiquées dans la revendication 1 et $R_5$ représente un groupe alkyle comportant de 1 à 4 atomes de carbone,
en présence d'une base forte et d'un solvant polaire.

**18.** Procédé de préparation de composés distyrylbiphényle symétriques de formule

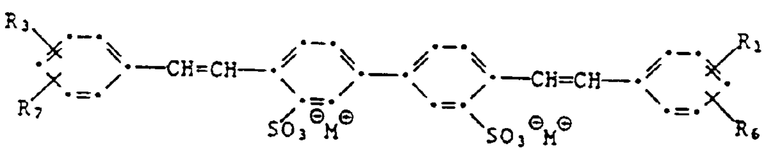 (III),

caractérisé en ce que l'on fait réagir deux moles d'un composé de formule (XX)

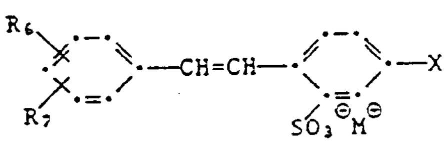 (XX)

en présence d'une base et de charbon palladié, dans un solvant polaire, $R_1$, $R_3$, $R_6$ et $R_7$ représentant chacun, indépendamment l'un de l'autre, un atome d'hydrogène ou d'halogène ou un groupe alkyle en $C_{1-4}$ ou CN, $M^+$ représentant un cation formant un sel et X représentant un atome d'halogène.

**19.** Utilisation des composés distyrylbiphényle conformes à la revendication 1 pour l'azurage optique de matériau textile.

**20.** Utilisation des composés distyrylbiphényle conformes à la revendication 1 pour l'azurage optique de coton, de polyamide et de laine.

21. Utilisation des composés distyrylbiphényle conformes à la revendication 1 pour l'azurage optique de papier.

22. Utilisation des composés distyrylbiphényle conformes à la revendication 1 dans des détergents solides, en tant qu'azurant optique.

23. Utilisation des composés distyrylbiphényle de formule

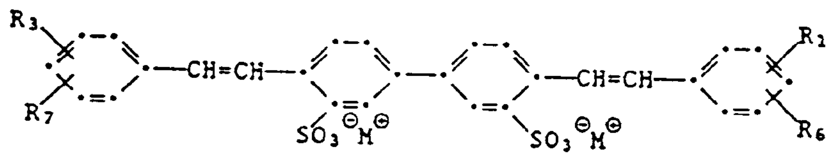

(III)

dans des détergents liquides, en tant qu'azurant optique, $R_1$, $R_3$, $R_6$ et $R_7$ représentant chacun, indépendamment l'un de l'autre, un atome d'hydrogène ou d'halogène ou un groupe alkyle en $C_{1-4}$ ou CN, et $M^+$ représentant un cation formant un sel.

24. Utilisation de composés distyrylbiphényle à substitution symétrique, de formule

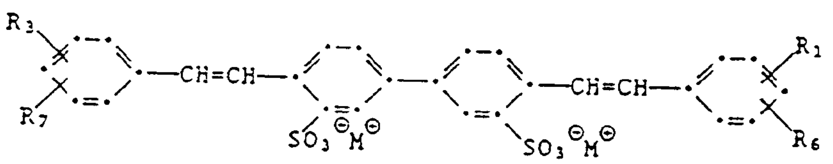

(III)

dans des détergents liquides, en tant qu'azurant optique, $R_1$, $R_3$, $R_6$ et $R_7$ représentant chacun, indépendamment l'un de l'autre, un atome d'hydrogène ou d'halogène ou un groupe alkyle en $C_{1-4}$ ou CN, et $M^+$ représentant un cation formant un sel.

25. Utilisation, selon la revendication 23, de composés distyrylbiphényle de formule

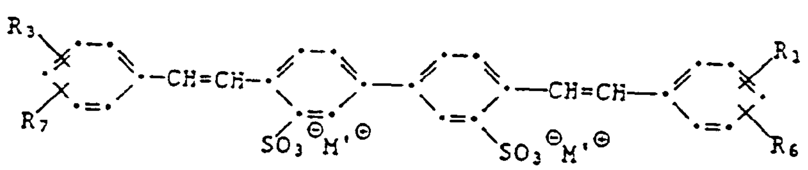

(IVa)

dans des détergents liquides, en tant qu'azurant optique, $M'^+$ représentant un ion de métal alcalin, un ion ammonium ou un ion d'amine et $R_1$, $R_3$, $R_6$ et $R_7$ ayant les significations indiquées dans la revendication 23.

26. Utilisation, selon la revendication 23, de composés distyrylbiphényle de formule

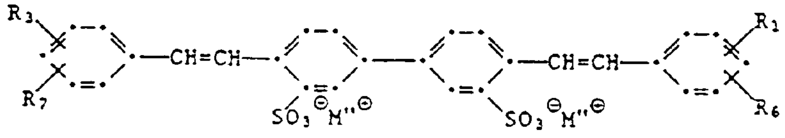

(Va)

dans des détergents liquides, en tant qu'azurant optique, $M''^{+}$ représentant un ion de sodium ou de potassium et $R_1$, $R_3$, $R_6$ et $R_7$ ayant les significations indiquées dans la revendication 23.

**27.** Utilisation, selon la revendication 23, de composés distyrylbiphényle de formule

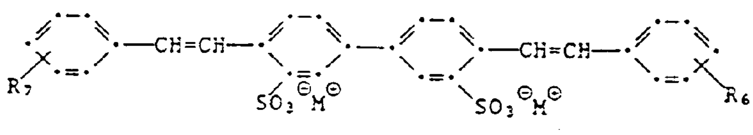

(VIa)

dans des détergents liquides, en tant qu'azurant optique, $R_6$, $R_7$ et $M^+$ ayant les significations indiquées dans la revendication 23.

**28.** Utilisation, selon la revendication 23, de composés distyrylbiphényle de formule

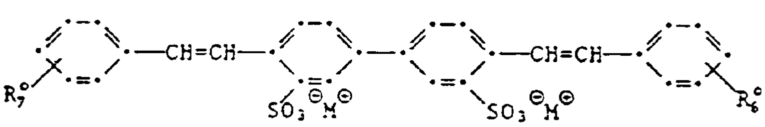

(VIIa)

dans des détergents liquides, en tant qu'azurant optique, $R^0{}_6$ et $R^0{}_7$ représentant un groupe alkyle en $C_{1-4}$ ou un atome de chlore et $M^+$ représentant un cation formant un sel.

**29.** Utilisation, selon la revendication 23, de composés distyrylbiphényle de formule

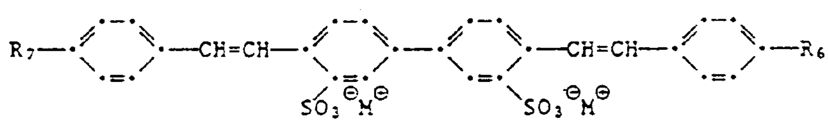

(IIa)

dans des détergents liquides, en tant qu'azurant optique, $R_6$, $R_7$ et $M^+$ ayant les significations indiquées dans la revendication 24.

**30.** Utilisation, selon la revendication 23, de composés distyrylbiphényle de formule

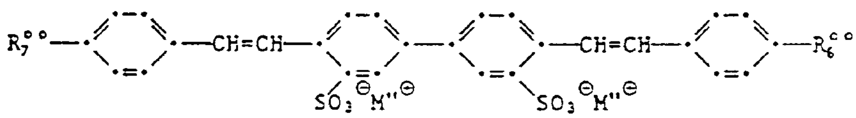

(VIIIa)

dans laquelle $R^{00}{}_6$ et $R^{00}{}_7$ représentent chacun un atome de chlore ou un groupe $CH_3$ et $M''^{+}$ représente un ion de sodium ou de potassium.